# EUROPEAN PATENT APPLICATION

(11) **EP 3 808 279 A1**
(43) Date of publication of application: **21.04.2021**
(21) Application number: 19202894.2
(22) Date of filing: 14.10.2019
(51) Int. Cl.: A61B 8/08, A61B 8/14, A61B 8/00, G01S 7/52

(54) **STEERABLE MULTI-PLANE ULTRASOUND IMAGING SYSTEM**

(71) Applicant: Koninklijke Philips N.V., 5656 AG Eindhoven (NL)
(72) Inventor: CHEN, Alvin, 5656 AE Eindhoven (NL); VAIDYA, Kunal, 5656 AE Eindhoven (NL); TORJESEN, Alyssa, 5656 AE Eindhoven (NL); LI, Sibo, 5656 AE Eindhoven (NL); ERKAMP, Ramon Quido, 5656 AE Eindhoven (NL); BHARAT, Shyam, 5656 AE Eindhoven (NL); FLEXMAN, Molly Lara, 5656 AE Eindhoven (NL); JAIN, Ameet Kumar, 5656 AE Eindhoven (NL); STAPERT, Hendrik Roelof, 5656 AE Eindhoven (NL); BUIL, Vincentius Paulus, 5656 AE Eindhoven (NL); CHEN, Nijn-Zu, 5656 AE Eindhoven (NL); MAESSEN, Ralph Theodorus Hubertus, 5656 AE Eindhoven (NL); NIJHOF, Niels, 5656 AE Eindhoven (NL); TALGORN, Elise Claude Valentine, 5656 AE Eindhoven (NL)
(74) Representative: Philips Intellectual Property & Standards

(57) **Abstract**

A steerable multi-plane ultrasound imaging system (MPUIS) for steering a plurality of intersecting image planes (PL_{1..n}) of a beamforming ultrasound imaging probe (BUIP) based on ultrasound signals transmitted between the beamforming ultrasound imaging probe (BUIP) and an ultrasound transducer (S) disposed within a field of view (FOV) of the probe (BUIP). An ultrasound tracking system (UTS) causes the beamforming ultrasound imaging probe (BUIP) to adjust an orientation of the first image plane (PL₁) such that a first image plane passes through a position (POS) of the ultrasound transducer (S) by maximizing a magnitude of ultrasound signals transmitted between the beamforming ultrasound imaging probe (BUIP) and the ultrasound transducer (S). An orientation of a second image plane (PL₂) is adjusted such that an intersection (AZ) between the first image plane and the second image plane passes through the position of the ultrasound transducer (S).

## Description

### FIELD OF THE INVENTION

The invention relates to a steerable multi-plane ultrasound imaging system. A related method and computer program product are also provided. The invention finds application in the medical ultrasound imaging field in particular and may be used with a variety of ultrasound imaging probes. Its use with transthoracic "TTE" ultrasound imaging probes, intravascular "IVUS", as well as transesophageal "TEE", transnasal "TNE", intracardiac "ICE", and transrectal "TRUS", ultrasound imaging probes, is contemplated.

### BACKGROUND OF THE INVENTION

A multi-plane ultrasound imaging system provides a medical practitioner with anatomical views to support a medical procedure. As compared to single plane ultrasound imaging, the additional views provided by a multi-plane imaging system provide improved visualization of the anatomy whilst avoiding the typically lower resolution or lower frame rates associated with full three-dimensional imaging.

In this respect, document US 2014/013849 A1 discloses a multi-plane ultrasound imaging system. Imaging data is acquired for a first plane and a second plane. The system includes adjusting a first orientation of the first plane and automatically adjusting a second orientation of the second plane in order to maintain a fixed relationship between the second plane and the first plane. Document US 2014/013849 A1 discloses to adjust the first plane by means of a user interface.

The invention addresses drawbacks of known multi-plane ultrasound imaging systems.

### SUMMARY OF THE INVENTION

The invention seeks to provide an improved multi-plane ultrasound imaging system. The invention is defined by the claims. Thereto, a steerable multi-plane ultrasound imaging system for steering a plurality of intersecting image planes of a beamforming ultrasound imaging probe based on ultrasound signals transmitted between the beamforming ultrasound imaging probe and an ultrasound transducer disposed within a field of view of the probe includes a beamforming ultrasound imaging probe and an ultrasound tracking system. The beamforming ultrasound imaging probe generates ultrasound beams that define a plurality of intersecting image planes, including a first image plane and a second image plane. The ultrasound tracking system causes the beamforming ultrasound imaging probe to adjust an orientation of the first image plane such that a first image plane passes through a position of the ultrasound transducer by maximizing a magnitude of ultrasound signals transmitted between the beamforming ultrasound imaging probe and the ultrasound transducer. The ultrasound tracking system also causes the beamforming ultrasound imaging probe to adjust an orientation of a second image plane such that an intersection between the first image plane and the second image plane passes through the position of the ultrasound transducer.

The position of the ultrasound transducer disposed is determined by maximizing a magnitude of ultrasound signals transmitted between the imaging probe and the transducer. The position then serves as a reference position through which an intersection of the image planes is caused to intersect. Tracking the ultrasound transducer position with the image planes in this manner compensates for relative movement between the imaging probe and objects within its field of view, which relative movement might otherwise cause the objects to disappear as they move out of the image plane(s). More stable planar images passing through the position are thus provided by the system, and without the drawbacks of lower resolution and/ or lower frame rates associated with three-dimensional image processing in which the entire three dimensional field of view is imaged. Further advantages of the described invention will also be apparent to the skilled person.

Further aspects are described with reference to the appended claims. Further advantages of these aspects will also be apparent to the skilled person.

### BRIEF DESCRIPTION OF THE FIGURES

Fig. 1 illustrates a steerable multi-plane ultrasound imaging system MPUIS that includes a beamforming ultrasound imaging probe BUIP with intersecting image planes PL₁, PL₂ within field of view FOV.
Fig. 2 illustrates in Fig. 2A - Fig. 2C the adjusting of image planes PL₁, PL₂ of a beamforming ultrasound imaging probe BUIP by tilting each image plane with respect to a normal axis NA.
Fig. 3 illustrates the adjusting of image planes PL₁, PL₂ of a beamforming ultrasound imaging probe BUIP based on an image feature detected in image planes PL₁.
Fig. 4 illustrates the reconstruction of a three-dimensional ultrasound image using ultrasound image data obtained whilst rotating image planes PL₁, PL₂.
Fig. 5 illustrates the generation of an overlay image in which a reconstructed ultrasound image is registered to an anatomical model AM, and the adjusting of image plane PL₂ to achieve a desired view defined in the anatomical model.
Fig. 6 illustrates a flowchart of a method MET that may be used in conjunction with some aspects of the disclosure.

### DETAILED DESCRIPTION OF THE INVENTION

In order to illustrate the principles of the present invention a steerable multi-plane ultrasound imaging system is described with particular reference to a beamforming ultrasound imaging probe in the form of a TTE probe. It is however to be appreciated that use of the system with alternative ultrasound imaging probes is also contemplated, including but not limited to IVUS, TEE, TNE, ICE, or TRUS, ultrasound imaging probes. Moreover, use of the system in combination with an interventional device is described with particular reference to the interventional device being a medical needle. It is however to be appreciated that the use of the system with other interventional devices is also contemplated, including but not limited to a catheter, a guidewire, a probe, an endoscope, an electrode, a robot, a filter device, a balloon device, a stent, a mitral clip, a left atrial appendage closure device, an aortic valve, a pacemaker, an intravenous line, a drainage line, a surgical tool, a tissue sealing device, a tissue cutting device or an implantable device.

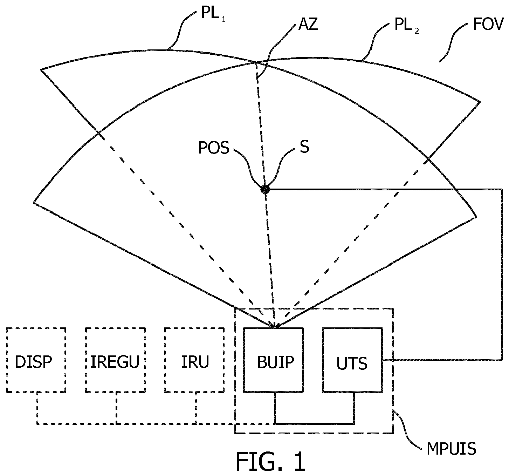

Thereto, Fig. 1 illustrates a steerable multi-plane ultrasound imaging system MPUIS that includes a beamforming ultrasound imaging probe BUIP with intersecting image planes PL₁, PL₂ within field of view FOV. Steerable multi-plane ultrasound imaging system MPUIS also includes ultrasound tracking system UTS, and may optionally include one or more of the illustrated units: image reconstruction unit IRU that generates a reconstructed ultrasound image corresponding to each of image planes PL₁, PL₂; image registration unit IREGU that generates an overlay image wherein the reconstructed ultrasound image is registered to an anatomical model; and display DISP that displays an image corresponding to each of image planes PL₁, PL₂ and/ or an anatomical model. The various units in Fig. 1 are in communication with each other as indicated by the connecting lines.

Steerable multi-plane ultrasound imaging system MPUIS in Fig. 1 is configured to generate and to steer multiple intersecting image planes, as exemplified by image planes PL₁, PL₂ of beamforming ultrasound imaging probe BUIP. As illustrated in Fig.1, image planes PL₁, PL₂ intersect transversely. In some implementations the planes intersect orthogonally. The image planes are each defined by a plurality of beams within which ultrasound signals, specifically ultrasound imaging signals, are transmitted and received. Image planes PL₁, PL₂ may be steered, i.e. their orientations may be adjusted, using beamsteering techniques known from the ultrasound field. Such techniques apply relative delays to the ultrasound imaging signals transmitted and received by elements of a two-dimensional array of ultrasound transducer elements of beamforming ultrasound imaging probe BUIP. Beamsteering techniques such as those disclosed in document US 2014/013849 A1 may for example be used. Beamforming ultrasound imaging probe BUIP may include or be controlled by electronic circuitry and/ or a processor in combination with a memory, which processor executes instructions stored in the memory and which instructions correspond to one or more of the aforementioned beam generation and steering techniques. Additional image planes to the two illustrated image planes PL₁, PL₂ may be provided and steered in a similar manner.

With reference to Fig. 1, an ultrasound transducer S is disposed within field of view FOV of beamforming ultrasound imaging probe BUIP. Field of view FOV represents the region within which beamforming ultrasound imaging probe BUIP may transmit and receive ultrasound imaging signals and thereby generate an ultrasound image. Ultrasound transducer S may be an ultrasound sensor, an ultrasound emitter or indeed capable of both sensing and emitting ultrasound signals. Disposing ultrasound transducer S within field of view FOV allows transducer S to receive ultrasound signals emitted by beamforming ultrasound imaging probe BUIP, and/ or vice versa allows beamforming ultrasound imaging probe BUIP to receive ultrasound signals emitted by transducer S. The use of piezoelectric transducers or Capacitive Micromachined Ultrasound Transducers, i.e. CMUT, transducers is contemplated for ultrasound transducer S. The use of hard and soft piezoelectric materials is contemplated. Polyvinylidene fluoride, otherwise known as PVDF whose mechanical properties and manufacturing processes lend themselves to attachment to curved surfaces such as medical needles may in particular be used. Alternative piezoelectric materials include a PVDF co-polymer such as polyvinylidene fluoride trifluoroethylene, a PVDF ter-polymer such as P(VDF-TrFE-CTFE). Other, non-piezoelectric materials may alternatively be used for ultrasound transducer S. In some implementations, ultrasound transducer S may be disposed on an interventional device, which may for example be a medical needle, or another interventional device. The interventional device may have an elongate axis. The ultrasound transducer may be wrapped around the elongate axis of the interventional device in order to provide ultrasound sensing and/ or emission around the elongate axis, although this is not essential.

Ultrasound tracking system UTS in Fig. 1 includes electronic circuitry and/ or a processor in combination with a memory, which processor executes instructions stored in the memory and which instructions correspond to the method steps of: causing beamforming ultrasound imaging probe BUIP to adjust an orientation of first image plane PL₁ such that the first image plane passes through a position of the ultrasound transducer S by maximizing a magnitude of ultrasound signals transmitted between the beamforming ultrasound imaging probe BUIP and ultrasound transducer S; and causing beamforming ultrasound imaging probe BUIP to adjust an orientation of the second image plane PL₂ such that an intersection AZ between the first image plane and the second image plane passes through the position of ultrasound transducer S.

Ultimately, image reconstruction unit IRU may generate a reconstructed ultrasound image corresponding to each of image planes PL₁, PL₂ and display DISP may display an image corresponding to each of image planes PL₁, PL₂.

In some implementations the reconstructed image may be displayed as a live image, and in other implementations the display of the reconstructed image may be synchronized to a particular cardiac or respiratory cycle and image data displayed only for a predetermined phase of the cycle. Such cardiac "gating" may for example be used to "freeze" the mitral valve in successive open or closed states, thereby allowing a medical practitioner to focus on this particular portion of the anatomy. The use of image-based segmentation, or cardiac/ respiratory sensor data received from a sensor such as an electrocardiogram sensor, i.e. ECG sensor, an ultrasound sensor, a strain sensor, a camera, or motion sensor and so forth are contemplated for determining the relevant cycle. A document "An open-source real-time ultrasound reconstruction system for four-dimensional imaging of moving organs" by Pace, D. et al (http://hdl.handle.net/10380/3083) provides an example of ECG gated 4D ultrasound for reconstructing 3D volumes. Thus, in this implementation, ultrasound tracking system UTS in Fig. 1 may include image reconstruction unit IRU that is configured to generate a reconstructed ultrasound image corresponding to each of image planes PL₁, PL₂, and display DISP that is configured to display an image corresponding to each of image planes PL₁, PL₂. The electronic circuitry and/ or the processor in combination with the memory of the ultrasound tracking system UTS in Fig. 1 may be further configured to execute instructions stored in the memory, which instructions correspond to the method steps of: receiving cardiac or respiratory cycle data corresponding to a subject within the field of view (FOV) of the probe (BUIP), identifying a predetermined phase within the cycle data, and gating the displaying of the reconstructed ultrasound image such that the image corresponding to each of image planes PL₁, PL₂ is displayed only at the predetermined phase of the cycle.

In operation, the orientations of the first image plane and the second image plane may be adjusted by for example tilting or rotating or translating the image plane. Beamforming ultrasound imaging probe BUIP may include a two-dimensional array of transducer elements having a normal axis NA, and adjusting an orientation of the first image plane PL₁ or the second image plane PL₂ may include at least one of: i) tilting the respective image plane PL₁, PL₂ with respect to the normal axis NA, ii) rotating the respective image plane PL₁, PL₂ about the normal axis NA, and iii) translating the respective image plane PL₁, PL₂ perpendicularly with respect to the normal axis NA.

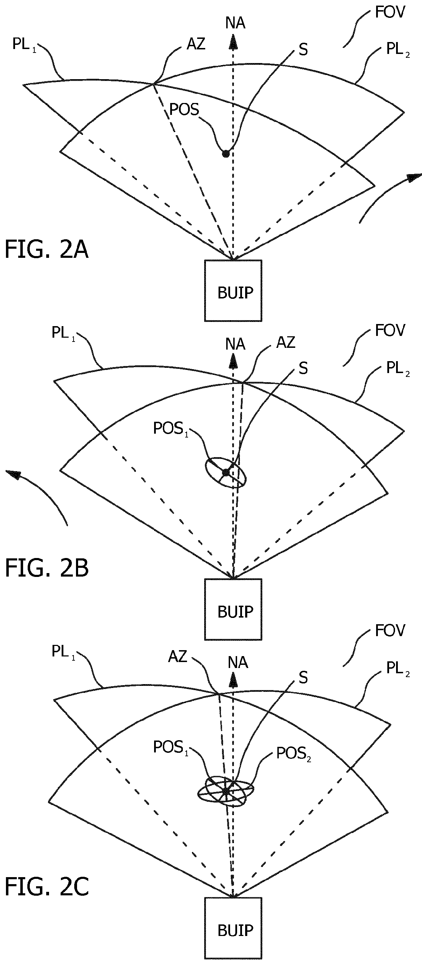

An example of an adjustment of image planes PL₁, PL₂ in accordance with the above method steps is shown in Fig. 2, which illustrates in Fig. 2A - Fig. 2C the adjusting of image planes PL₁, PL₂ of a beamforming ultrasound imaging probe BUIP by tilting each image plane with respect to a normal axis NA. In Fig. 2A, an intersection AZ between first image plane PL₁ and second image plane PL₂ initially does not pass through position POS of ultrasound transducer S. This may be considered to represent an initial arrangement prior to tracking the positon of transducer S. As indicated by the arrow in Fig. 2A, image plane PL₁ is then adjusted, by tilting, until, and as indicated in Fig. 2B, first image plane PL₁ passes through the position of ultrasound transducer S. This is achieved by maximizing a magnitude of ultrasound signals transmitted between beamforming ultrasound imaging probe BUIP and ultrasound transducer S. As indicated in Fig. 2B, an in-plane position POS₁ on first image plane PL₁, is thus identified. In order to cause intersection AZ between first image plane PL₁ and second image plane PL₂ to pass through the position of ultrasound transducer, image plane PL₂ is tilted, as indicated by the arrow in Fig. 2B, to provide the arrangement indicated in Fig. 2C, wherein intersection AZ passes through the position of ultrasound transducer. As indicated in Fig. 2C, an in-plane position POS₂ on second image plane PL₂, may thus be identified, POS₂ being coincident with POS₁.

Subsequently, a magnitude of ultrasound signals transmitted between the beamforming ultrasound imaging probe BUIP and ultrasound transducer S is measured continually and image planes PL₁, PL₂ are adjusted continually in the same manner such that the intersection of image planes PL₁, PL₂ continues to intersect subsequent positions of ultrasound transducer S.

In some implementations ultrasound transducer S is an ultrasound sensor, and in other implementations ultrasound transducer S is an ultrasound emitter. Moreover, the ultrasound signals may be ultrasound imaging signals transmitted by beamforming ultrasound imaging probe, or dedicated ultrasound tracking signals that are not used for imaging purposes. The tracking signals may be directional beams emitted by beamforming ultrasound imaging probe BUIP within field of view FOV, or omnidirectional signals emitted by transducer S. In some implementations a plurality of ultrasound emitters or receivers are disposed on beamforming ultrasound imaging probe BUIP and ultrasound tracking signals are respectively transmitted or received by these emitters or receivers.

Thus, the following are contemplated in this respect: i) ultrasound transducer S is an ultrasound sensor, and the ultrasound signals are ultrasound imaging signals transmitted by beamforming ultrasound imaging probe BUIP and received by ultrasound sensor S; ii) ultrasound transducer S is an ultrasound sensor, and the ultrasound signals are ultrasound tracking signals transmitted by beamforming ultrasound imaging probe BUIP, said ultrasound tracking signals being interleaved between ultrasound imaging signals, and said ultrasound tracking signals being received by ultrasound sensor S; or iii) ultrasound transducer S is an ultrasound sensor, and the ultrasound signals are ultrasound tracking signals transmitted by each of a plurality of ultrasound emitters disposed on the beamforming ultrasound imaging probe BUIP, said ultrasound tracking signals being received by ultrasound sensor S; or iv) ultrasound transducer S is an ultrasound emitter, and the ultrasound signals are transmitted by the ultrasound emitter and received by beamforming ultrasound imaging probe BUIP; or iv) ultrasound transducer S is an ultrasound emitter, and the ultrasound signals are transmitted by the ultrasound emitter and received by each of a plurality of ultrasound receivers disposed on beamforming ultrasound imaging probe BUIP.

The method step of causing beamforming ultrasound imaging probe BUIP to adjust an orientation of the second image plane PL₂ such that an intersection AZ between the first image plane and the second image plane passes through the position of the ultrasound transducer S may be carried out simultaneously with, or after, the method step of adjusting an orientation of first image plane PL₁. This may be achieved based on the position POS of ultrasound transducer S.

In some implementations first image plane PL₁ and second image plane PL₂ are adjusted by:
adjusting first image plane PL₁ and second image plane PL₂ simultaneously such that the maximum generated electrical signal on the first image plane is maximized; and
adjusting second image plane PL₂ independently of first image plane PL₁ such that the maximum generated electrical signal on the second image plane PL₂ is maximized.

After image planes PL₁, PL₂ have been adjusted such that intersection AZ passes through the position of ultrasound transducer S, ultrasound tracking system UTS may thus continue to track movements of ultrasound transducer S to each of a plurality of new positions by adjusting an orientation of first image plane PL₁ and second image plane PL₂ such that intersection AZ between first image plane PL₁ and second image plane PL₂ passes through each new position of ultrasound transducer S. In order to do this, first image plane PL₁ and second image plane PL₂ may each be alternately adjusted, i.e. dithered, in opposing directions transversely with respect to their respective plane in order to search for a new position in which the magnitude of ultrasound signals transmitted between beamforming ultrasound imaging probe BUIP and the ultrasound transducer S is maximal. Such adjustments may be made continually, periodically, or in response to a change in the magnitude of the ultrasound signals transmitted between beamforming ultrasound imaging probe BUIP and ultrasound transducer S.

In some implementations, ultrasound tracking system UTS may track movements of ultrasound transducer S to each of a plurality of new positions by adjusting an orientation of first image plane PL₁ and second image plane PL₂ such that intersection AZ between first image plane PL₁ and second image plane PL₂ passes through each new position of ultrasound transducer S, and if the magnitude of the ultrasound signals transmitted between beamforming ultrasound imaging probe BUIP and ultrasound transducer S falls below a predetermined threshold value, which threshold value may for example be indicative of an unreliable position, or of ultrasound transducer having moved to an out-of-plane position too quickly to be tracked, ultrasound tracking system UTS may further cause beamforming ultrasound imaging probe BUIP to repeat the steps of:
adjusting an orientation of first image plane PL₁ such that the first image plane passes through a position of ultrasound transducer S by maximizing a magnitude of ultrasound signals transmitted between the beamforming ultrasound imaging probe BUIP and ultrasound transducer S; and
causing beamforming ultrasound imaging probe BUIP to adjust an orientation of second image plane PL₂ such that an intersection AZ between the first image plane and the second image plane passes through the position of the ultrasound transducer S.

Tracking position POS of ultrasound transducer S with image planes PL₁, PL₂ in this manner provides planar images that pass through position POS and also compensate for relative movements between the beamforming ultrasound imaging probe BUIP and an object within field of view FOV. Thus, more stable planar images passing through the position are thus provided without the drawbacks of lower resolution and/ or lower frame rates associated with three-dimensional image processing in which the entire three dimensional field of view is imaged.

In some implementations the ultrasound tracking system UTS in Fig. 1 may identify a maximum signal ultrasound beam Bₘₐₓ for the first image plane PL₁. The maximum signal ultrasound beam Bₘₐₓ is defined as the ultrasound beam for which the magnitude of ultrasound signals transmitted between the beamforming ultrasound imaging probe BUIP and the ultrasound transducer S is the highest for first image plane PL₁. In this implementation, causing beamforming ultrasound imaging probe BUIP to adjust the second image plane PL₂ such that the intersection AZ between the first image plane and the second image plane passes through the position of the ultrasound transducer S may include causing the second image plane PL₂ to intersect the maximum signal ultrasound beam Bₘₐₓ. When the ultrasound tracking system uses ultrasound imaging beams, the maximum signal ultrasound beam Bₘₐₓ can be readily identified. This beam thus provides an easy reference beam to which the second image plane PL₂ may be aligned.

For example, in implementations in which ultrasound transducer S is an ultrasound sensor, and wherein ultrasound signals are transmitted by beamforming ultrasound imaging probe BUIP and received by ultrasound sensor S, ultrasound tracking system UTS may be further configured to:
receive electrical signals generated by ultrasound sensor S in response to the ultrasound signals transmitted by the beamforming ultrasound imaging probe BUIP;
receive synchronization signals from beamforming ultrasound imaging probe BUIP, the synchronization signals corresponding to a time of emission of the transmitted ultrasound signals; and to
identify the maximum signal ultrasound beam Bₘₐₓ based on the received electrical signals and the received synchronization signals.

The synchronization signals identify each beam that is transmitted by beamforming ultrasound imaging probe BUIP. The magnitudes of the electrical signals generated in response to each transmitted beam are compared for first image plane PL₁ to determine the beam in which the generated electrical signal is maximum. The beam in which the generated electrical signal is maximum, identifies the beam that is closest to the position of sensor S. This beam defines an in-plane angle of sensor S with respect to the plane. Optionally, a time of flight corresponding to the time difference between the time of generation of the maximum electrical signal and the time of transmission of the ultrasound signals that gave rise to the maximum electrical signal, may additionally be computed in order to determine a distance, i.e. a range, between sensor S and beamforming ultrasound imaging probe BUIP. This procedure results in the determination of a beam in which sensor S is disposed, and/ or a range of sensor S.

In an alternative ultrasound tracking system UTS, which may be used particularly in implementations having a plurality of ultrasound emitters or receivers disposed on beamforming ultrasound imaging probe BUIP, triangulation may be used to determine a position of ultrasound transducer S in relation to beamforming ultrasound imaging probe BUIP. Such a tracking system, often termed a sono-micrommetry system, may determine the distances between each ultrasound emitter/ receiver disposed on beamforming ultrasound imaging probe BUIP and transducer S from the time of flight of ultrasound signals between the respective emitter/ receiver and ultrasound transducer S. Using triangulation and the speed of propagation of ultrasound signals, the distances between ultrasound transducer S and at least three emitters/ receivers may be used to determine position POS of ultrasound transducer S in terms of a relative angle, and optionally additionally a distance, i.e. a range, between beamforming ultrasound imaging probe BUIP and ultrasound transducer S.

After image planes PL₁, PL₂ have been adjusted such that intersection AZ passes through the position of ultrasound transducer S, in with some implementations, ultrasound tracking system UTS may further cause beamforming ultrasound imaging probe BUIP to adjust at least one of the first image plane PL₁ and the second image plane PL₂ based on an image feature. The image feature may be detected in the respective plane, for example using known image segmentation techniques or known model-fitting techniques such as feature based object segmentation or 3D augmented model registration. A document entitled "3D Ultrasound image segmentation: A Survey" by Mozaffari, M. H., and Lee, W. https://arxiv.org/abs/1611.09811 discloses some suitable techniques. At the same time it is maintained that the intersection AZ between the first image plane and the second image plane passes through the position of the ultrasound transducer S.

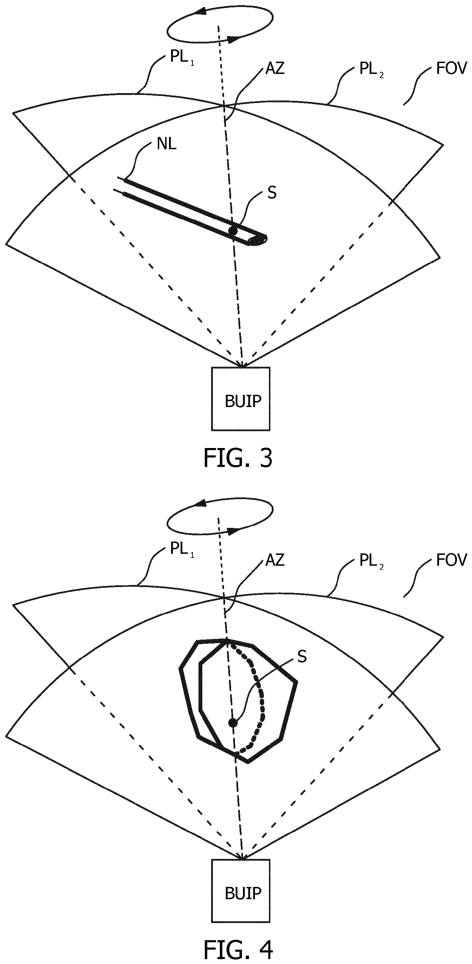

This is illustrated in Fig. 3 which illustrates the adjusting of image planes PL₁, PL₂ of a beamforming ultrasound imaging probe BUIP based on an image feature detected in image plane PL₁. In Fig. 3 the image feature is a medical needle NL which is segmented in image plane PL₁. The orientation of image plane PL₁ is adjusted, in the present example by rotating image plane PL₁ in order to maximize the segmented area of medical needle NL by rotating image plane PL₁ such that it is parallel to and passes through the longitudinal axis of medical needle NL. In an alternative implementation, image plane PL₁ may be rotated such that it passes perpendicularly through the longitudinal axis of medical needle NL. The image plane(s) may thus be adjusted by maximizing the correspondence between an expected image shape and a shape segmented in the image plane. In the case of the shape being a medical needle, the image plane may be rotated until the segmented shape becomes as close as possible to a circle or a straight line; a circle and a straight line being orthogonal cross sectional shapes of the medical needle. Other angles of intersection between the longitudinal axis of medical needle NL and image plane PL₁ may likewise be provided in a similar manner by rotating image plane PL₁ until a target cross sectional shape is provided by the segmentation, thereafter making adjustments to image plane PL₁ to maintain the target cross sectional shape. Image plane PL₂, and indeed any other image planes not shown in Fig. 3, may likewise be rotated in order to either maintain a constant mutual angular relationship with image plane PL₁ with respect to intersection AZ, or their image planes may remain un-adjusted.

The terms parallel and perpendicular as used herein refer to within ± 5 degrees of exactly parallel and exactly perpendicular.

By maintaining this tracking, and also providing an image plane based on the image feature, a desired view may be provided. The image feature may in general be a portion of the anatomy, or a portion of an interventional device to which the ultrasound transducer is attached, or a portion of a second interventional device within the field of view of the beamforming ultrasound imaging probe.

In some implementations, image planes PL₁, PL₂, and any additional image planes that may exist, may be adjusted simultaneously in response to movements of the image feature whilst maintaining a constant angle of intersection. This advantageously allows for e.g. the tracking of an anatomical feature whilst maintaining the intersection of the image planes at a reference point, specifically the position of ultrasound transducer S. The selection of the image feature may in some instances be determined based on user input, for example based on user input received from a user interface comprising a menu of image features or based on input in the form of a user selection of a portion of the reconstructed image corresponding to image plane PL₁.

In one exemplary implementation the at least one of the first image plane PL₁ and the second image plane PL₂ may be adjusted based on the image feature by: computing a value of an image quality metric corresponding to the image feature; and adjusting the at least one the first image plane PL₁ and the second image plane PL₂ to maximize the value of the image quality metric.

The image quality metric may for instance be i) a completeness of a segmentation of the image feature in the respective image plane PL₁, PL₂ or ii) a closeness of a fit of the segmentation to a model to the image feature. For example, if the image feature is a portion of the aortic valve, the image quality metric may represent the completeness, i.e. the intensity and/ or the contiguity of the pixels of a segmented annular image feature corresponding to the aortic valve. The annular feature here serves as a model of the desired anatomical region. By maximizing the completeness of the segmentation, the orientation of the first image plane may be continually or periodically adjusted to maintain the most-complete image of the aortic valve in the first image plane. This feature may prove beneficial in applications such as TAVI (Trans-catheter aortic valve implantation) and other structural interventions. This advantageously prevents that the user has to continually adjust the positioning of the imaging probe in order to achieve a desired view.

With reference to Fig. 4 and to Fig. 1, in some implementations, after image planes PL₁, PL₂ have been adjusted such that intersection AZ passes through the position of ultrasound transducer S, image reconstruction unit IRU may reconstruct a three-dimensional ultrasound image by rotating one or more of the image planes PL_{1..n} whilst maintaining the intersection of the image planes with the position of the ultrasound transducer. This is illustrated in Fig. 4, which illustrates the reconstruction of a three-dimensional ultrasound image using ultrasound image data obtained whilst rotating image planes PL₁ and PL₂. In Fig. 4, beamforming ultrasound imaging probe BUIP, which may be used in place of the same-referenced item in Fig. 1, is illustrated as generating image data for each of image planes PL₁, PL₂ by means of the thick solid lines for each image plane. Whilst maintaining that intersection AZ passes through the position of sensor S, both image planes are rotated through 90 degrees about intersection AZ and image data at each of a plurality of rotational angles is generated and recorded. The image data is then rendered into a three-dimensional image. Alternatively, data from only one plane, such as image plane PL₁, may be provided and used in such a three-dimensional image reconstruction. For example, data may be recorded and rendered for image plane PL₁ whilst rotating the plane through 180 degrees, or through another angle. The use of other numbers of image planes and other rotational angles than these examples is also contemplated in this respect. In some implementations some overlap in the image data generated at the start and the end of the rotation may be desirable in order to provide redundant overlapping image data in order to match the image data obtained at the start and the ends of the rotation. Thus angles slightly larger than 360°/2*n* may be used in some implementations, wherein n is the number of image planes.

Thus, in such implementations, steerable multi-plane ultrasound imaging system MPUIS in Fig. 1 also includes image reconstruction unit IRU that reconstruct ultrasound images based on ultrasound image data generated by the beamforming ultrasound imaging probe BUIP for each of a plurality of image planes such as image planes PL₁, PL₂. Ultrasound tracking system UTS may also cause beamforming ultrasound imaging probe BUIP to adjust one or more of image planes PL₁, PL₂ by rotating the image plane(s) about the intersection AZ between the first image plane PL₁ and the second image plane PL₂, and to reconstruct a three-dimensional ultrasound image based on ultrasound image data corresponding to at least one of the plurality of intersecting image planes during the rotation.

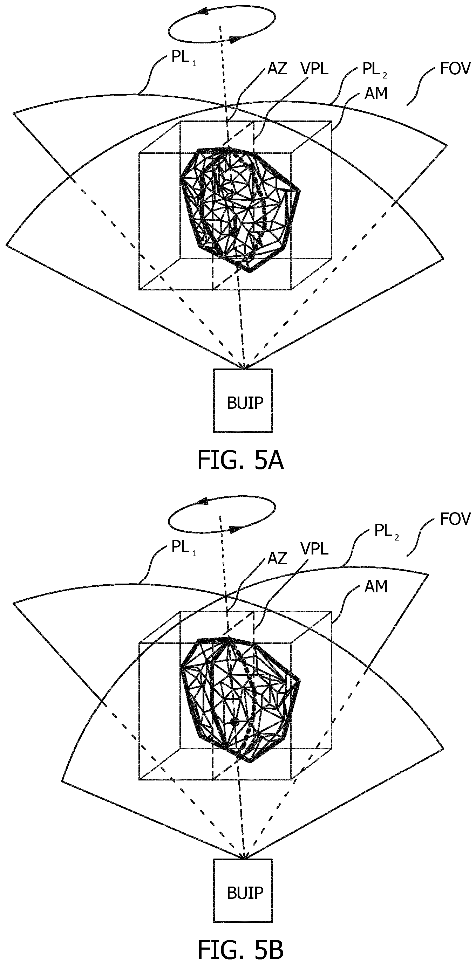

With reference to Fig. 5 and to Fig. 1, in some implementations, after image planes PL₁, PL₂ have been adjusted such that intersection AZ passes through the position of ultrasound transducer S, at least one of image planes PL₁, PL₂ may be adjusted so as to provide a desired view defined in an anatomical model. In these implementations, steerable multi-plane ultrasound imaging system MPUIS in Fig. 1 may include an image reconstruction unit IRU and an image registration unit IREGU that generates an overlay image wherein reconstructed ultrasound images are registered to an anatomical model. In Fig. 5, beamforming ultrasound imaging probe BUIP, which may be used in place of the same-referenced item in Fig. 1, is illustrated as generating image data for each of image planes PL₁, PL₂ by means of the thick solid lines for each image plane. Fig. 5 also includes an anatomical model AM, by means of the segmented structure within the cubic reference frame, which model corresponds to an anatomical region within field of view FOV. Anatomical model AM may be stored in a memory comprising a library of anatomical models that are selectable based on user input. Ultrasound images from one or more of image planes PL₁, PL₂ are registered to anatomical model AM. Image registration unit IREGU generates an overlay image in which the reconstructed ultrasound image(s) are registered to the anatomical model. As illustrated in the differences between Fig. 5A and Fig. 5B, the ultrasound tracking system UTS then causes beamforming ultrasound imaging probe BUIP to adjust image plane PL₁ in order to achieve a desired view defined in the anatomical model.

In more detail, in Fig. 5A, anatomical model AM, i.e. the segmented structure within the cubic reference frame, includes a visualization plane VPL as indicated by the plane with dashed lines. Visualization plane VPL may for example correspond to a desired image slice through the anatomy. An example of such a slice could be the annular plane used to visualize the mitral valve and the annulus during a mitral clip procedure. Visualization plane VPL may be selected by means of user input received via user input device - for example a user selecting a plane, i.e. a desired view on an image of the anatomical model. Ultrasound tracking system UTS causes beamforming ultrasound imaging probe BUIP to provide the desired view VPL by rotating one or more of image planes PL₁, PL₂ about the intersection AZ of the first image plane PL₁ and the second image plane PL₂ such that, one of the planes, in this example, image plane PL₂ is parallel to visualization plane VPL. Whilst in Fig. 5, only image plane PL₂ is rotated, and image plane PL₁ remains un-adjusted, both image planes PL₁ and PL₂ may alternatively be caused to rotate such that one of the planes is parallel to visualization plane VPL. The planes may thus be rotated such that they maintain a constant mutual angular relationship one another with one another with respect to intersection AZ, or alternatively only one of image planes PL₁, PL₂ may be rotated. One or more additional visualization planes to image planes VPL may also be defined on the model, and other image planes from image planes PL_{1..n} of steerable multi-plane ultrasound imaging system MPUIS may be caused to rotate independently to provide these additional visualization plane(s).

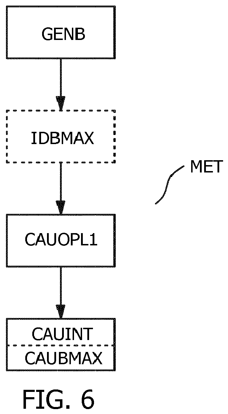

Fig. 6 illustrates a flowchart of a method MET that may be used in conjunction with some aspects of the disclosure. Method MET may be used to steer a plurality of intersecting image planes PL_{1..n} of a beamforming ultrasound imaging probe BUIP based on ultrasound signals transmitted between the beamforming ultrasound imaging probe BUIP and an ultrasound transducer S disposed within a field of view FOV of the probe BUIP. Method MET may in particular be used in any of the systems described with reference to Fig. 1 -Fig.5. Method MET includes the steps of:
generating GENB a plurality of ultrasound beams to define a plurality of intersecting image planes PL_{1..n}, the image planes comprising at least a first image plane PL₁ and a second image plane PL₂;
causing CAUOPL1 the beamforming ultrasound imaging probe BUIP to adjust an orientation of the first image plane PL₁ such that the first image plane passes through a position of the ultrasound transducer S by maximizing a magnitude of ultrasound signals transmitted between the beamforming ultrasound imaging probe BUIP and the ultrasound transducer S;
causing CAUINT the beamforming ultrasound imaging probe BUIP to adjust an orientation of the second image plane PL₂ such that an intersection AZ between the first image plane and the second image plane passes through the position of the ultrasound transducer S.

The method may in particular be used in configurations wherein the ultrasound transducer S is an ultrasound sensor, and wherein the ultrasound signals are transmitted by the beamforming ultrasound imaging probe BUIP and received by the ultrasound sensor S. In such configurations the method may further include the steps of:
identifying IDBMAX a maximum signal ultrasound beam Bₘₐₓ for the first image plane PL₁, the maximum signal ultrasound beam Bₘₐₓ being an ultrasound beam for which the magnitude of ultrasound signals transmitted between the beamforming ultrasound imaging probe BUIP and the ultrasound transducer S is the highest for the first image plane PL₁; and
the step of:
causing the beamforming ultrasound imaging probe BUIP to adjust the second image plane PL₂ such that an intersection AZ between the first image plane and the second image plane passes through the position of the ultrasound transducer S may further comprise:
the step of:
causing CAUBMAX the second image plane PL₂ to intersect the maximum signal ultrasound beam Bₘₐₓ.

Moreover, one or more additional steps disclosed in connection with system MPUIS may also be included in method MET.

Any of the method steps disclosed herein may be recorded in the form of instructions which when executed on a processor cause the processor to carry out such method steps. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor "DSP" hardware, read only memory "ROM" for storing software, random access memory "RAM", non-volatile storage, etc. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray™ and DVD.

In summary, a steerable multi-plane ultrasound imaging system for steering a plurality of intersecting image planes of a beamforming ultrasound imaging probe based on ultrasound signals transmitted between the beamforming ultrasound imaging probe and an ultrasound transducer disposed within a field of view of the probe has been described. An ultrasound tracking system causes the beamforming ultrasound imaging probe to adjust an orientation of the first image plane such that a first image plane passes through a position of the ultrasound transducer by maximizing a magnitude of ultrasound signals transmitted between the beamforming ultrasound imaging probe and the ultrasound transducer. An orientation of a second image plane is adjusted such that an intersection between the first image plane and the second image plane passes through the position of the ultrasound transducer.

Various embodiments and options have been described in relation to the system, and it is noted that the various embodiments may be combined to achieve further advantageous effects. Any reference signs in the claims should not be construed as limiting the scope of the invention.

## Claims

1. A steerable multi-plane ultrasound imaging system (MPUIS) for steering a plurality of intersecting image planes (PL_{1..n}) of a beamforming ultrasound imaging probe (BUIP) based on ultrasound signals transmitted between the beamforming ultrasound imaging probe (BUIP) and an ultrasound transducer (S) disposed within a field of view (FOV) of the probe (BUIP), the system comprising:
a beamforming ultrasound imaging probe (BUIP); and
an ultrasound tracking system (UTS);
wherein the beamforming ultrasound imaging probe (BUIP) is configured to generate ultrasound beams that define a plurality of intersecting image planes (PL_{1..n}), the image planes comprising at least a first image plane (PL₁) and a second image plane (PL₂);
wherein the ultrasound tracking system (UTS) is in communication with the beamforming ultrasound imaging probe (BUIP) and is configured to cause the beamforming ultrasound imaging probe (BUIP) to adjust an orientation of the first image plane (PL₁) such that the first image plane passes through a position (POS) of the ultrasound transducer (S) by maximizing a magnitude of ultrasound signals transmitted between the beamforming ultrasound imaging probe (BUIP) and the ultrasound transducer (S); and to
cause the beamforming ultrasound imaging probe (BUIP) to adjust an orientation of the second image plane (PL₂) such that an intersection (AZ) between the first image plane and the second image plane passes through the position of the ultrasound transducer (S).

2. The system according to claim 1 wherein i) the ultrasound transducer (S) is an ultrasound sensor, and wherein the ultrasound signals are ultrasound imaging signals transmitted by the beamforming ultrasound imaging probe (BUIP) and received by the ultrasound sensor (S); or ii) the ultrasound transducer (S) is an ultrasound sensor, and wherein the ultrasound signals are ultrasound tracking signals transmitted by the beamforming ultrasound imaging probe (BUIP), said ultrasound tracking signals being interleaved between ultrasound imaging signals, and said ultrasound tracking signals being received by the ultrasound sensor (S); or wherein iii) the ultrasound transducer (S) is an ultrasound sensor, and wherein the ultrasound signals are ultrasound tracking signals transmitted by each of a plurality of ultrasound emitters disposed on the beamforming ultrasound imaging probe (BUIP), said ultrasound tracking signals being received by the ultrasound sensor (S); or wherein iv) the ultrasound transducer (S) is an ultrasound emitter, and wherein the ultrasound signals are transmitted by the ultrasound emitter and received by the beamforming ultrasound imaging probe (BUIP); or wherein iv) the ultrasound transducer (S) is an ultrasound emitter, and wherein the ultrasound signals are transmitted by the ultrasound emitter and received by each of a plurality of ultrasound receivers disposed on the beamforming ultrasound imaging probe (BUIP).

3. The system according to claim 1 wherein the ultrasound tracking system (UTS) is further configured to identify a maximum signal ultrasound beam (Bₘₐₓ) for the first image plane (PLi), the maximum signal ultrasound beam (Bₘₐₓ) being an ultrasound beam for which the magnitude of ultrasound signals transmitted between the beamforming ultrasound imaging probe (BUIP) and the ultrasound transducer (S) is the highest for the first image plane PL₁; and
wherein causing the beamforming ultrasound imaging probe (BUIP) to adjust the second image plane (PL₂) such that an intersection (AZ) between the first image plane and the second image plane passes through the position of the ultrasound transducer (S) comprises causing the second image plane (PL₂) to intersect the maximum signal ultrasound beam (Bₘₐₓ).

4. The system according to claim 3 wherein the ultrasound transducer (S) is an ultrasound sensor, and wherein the ultrasound signals are transmitted by the beamforming ultrasound imaging probe (BUIP) and received by the ultrasound sensor (S); and wherein the ultrasound tracking system (UTS) is further configured to:
receive electrical signals generated by the ultrasound sensor (S) in response to the ultrasound signals transmitted by the beamforming ultrasound imaging probe (BUIP);
receive synchronization signals from the beamforming ultrasound imaging probe (BUIP), the synchronization signals corresponding to a time of emission of the transmitted ultrasound signals; and to
identify the maximum signal ultrasound beam (Bₘₐₓ) based on the received electrical signals and the received synchronization signals.

5. The system according to claim 1 wherein the beamforming ultrasound imaging probe (BUIP) comprises a two-dimensional array of transducer elements having a normal axis (NA), and wherein adjusting an orientation of the first image plane (PL₁) or the second image plane (PL₂) comprises at least one of: i) tilting the respective image plane (PL₁, PL₂) with respect to the normal axis (NA), ii) rotating the respective image plane (PL₁, PL₂) about the normal axis (NA), and iii) translating the respective image plane (PL₁, PL₂) perpendicularly with respect to the normal axis (NA).

6. The system according to claim 1 wherein the ultrasound tracking system (UTS) is further configured to track movements of the ultrasound transducer (S) to each of a plurality of new positions by adjusting an orientation of at least the first image plane (PL1) and the second image plane (PL2) such that the intersection (AZ) between the first image plane (PL1) and the second image plane (PL2) passes through each new position of the ultrasound transducer (S); and wherein
if the magnitude of the ultrasound signals transmitted between the beamforming ultrasound imaging probe (BUIP) and the ultrasound transducer (S) falls below a predetermined threshold value, the ultrasound tracking system (UTS) is further configured to cause the beamforming ultrasound imaging probe (BUIP) to repeat the steps of:
adjusting an orientation of the first image plane (PL₁) such that the first image plane passes through a position of the ultrasound transducer (S) by maximizing a magnitude of ultrasound signals transmitted between the beamforming ultrasound imaging probe (BUIP) and the ultrasound transducer (S); and
causing the beamforming ultrasound imaging probe (BUIP) to adjust an orientation of the second image plane (PL₂) such that an intersection (AZ) between the first image plane and the second image plane passes through the position of the ultrasound transducer (S).

7. The system according to claim 1 wherein the first image plane (PL₁) and the second image plane (PL₂) are adjusted by:
adjusting the first image plane (PL₁) and the second image plane (PL₂) simultaneously such that the maximum generated electrical signal on the first image plane is maximized; and
adjusting the second image plane (PL₂) independently of the first image plane (PL₁) such that the maximum generated electrical signal on the second image plane (PL₂) is maximized.

8. The system according to claim 1 wherein the ultrasound tracking system (UTS) is further configured to cause the beamforming ultrasound imaging probe (BUIP) to adjust at least one of the first image plane (PL₁) and the second image plane (PL₂) based on an image feature detected in the respective image plane (PL₁, PL₂); whilst maintaining that the intersection (AZ) between the first image plane and the second image plane passes through the position of the ultrasound transducer (S).

9. The system according to claim 8 wherein the ultrasound tracking system (UTS) is configured to cause the beamforming ultrasound imaging probe (BUIP) to adjust the at least one of the first image plane (PL₁) and the second image plane (PL₂) based on the image feature by:
computing a value of an image quality metric corresponding to the image feature; and
adjusting the at least one the first image plane (PL₁) and the second image plane (PL₂) to maximize the value of the image quality metric.

10. The system according to claim 9 wherein computing the image quality metric comprises i) segmenting the image feature in the respective image plane (PL₁, PL₂) or ii) fitting a model to the image feature.

11. The system according to claim 1 further comprising an image reconstruction unit (IRU) configured to reconstruct ultrasound images based on ultrasound image data generated by the beamforming ultrasound imaging probe (BUIP) for each of the image planes (PL_{1..n}), and wherein the ultrasound tracking system (UTS) is further configured to cause the beamforming ultrasound imaging probe (BUIP) to adjust the plurality of image planes (PL_{1..n}) by rotating the plurality of image planes (PL_{1..n}) about the intersection (AZ) between the first image plane and the second image plane, and to reconstruct a three-dimensional ultrasound image based on ultrasound image data corresponding to at least one of the plurality of intersecting image planes (PL_{1..n}) during said rotation.

12. The system according to claim 1 further comprising an image reconstruction unit (IRU) configured to reconstruct ultrasound images based on ultrasound image data generated by the beamforming ultrasound imaging probe for each of the image planes (PL_{1..n});
and further comprising an image registration unit (IREGU) configured to generate an overlay image wherein the reconstructed ultrasound images are registered to an anatomical model (AM); and
wherein the ultrasound tracking system (UTS) is configured to cause the beamforming ultrasound imaging probe (BUIP) to adjust at least one of the image planes (PL_{1..n}) based on a desired view defined in the anatomical model (AM).

13. The system according to claim 12 wherein the desired view comprises a visualization plane (VPL); and wherein the ultrasound tracking system (UTS) is configured to cause the beamforming ultrasound imaging probe (BUIP) to provide the desired view (VPL) by rotating the at least one of the image planes about the intersection (AZ) of the first image plane (PL₁) and the second image plane (PL₂) such that the at least one of the image planes is parallel to the visualization plane.

14. Method (MET) of steering a plurality of intersecting image planes (PL_{1..n}) of a beamforming ultrasound imaging probe (BUIP) based on ultrasound signals transmitted between the beamforming ultrasound imaging probe (BUIP) and an ultrasound transducer (S) disposed within a field of view (FOV) of the probe (BUIP), the method comprising the steps of:
generating a plurality of ultrasound beams to define a plurality of intersecting image planes (PL_{1..n}), the image planes comprising at least a first image plane (PL₁) and a second image plane (PL₂);
causing the beamforming ultrasound imaging probe (BUIP) to adjust an orientation of the first image plane (PL₁) such that the first image plane passes through a position of the ultrasound transducer (S) by maximizing a magnitude of ultrasound signals transmitted between the beamforming ultrasound imaging probe (BUIP) and the ultrasound transducer (S);
causing the beamforming ultrasound imaging probe (BUIP) to adjust an orientation of the second image plane (PL₂) such that an intersection (AZ) between the first image plane and the second image plane passes through the position of the ultrasound transducer (S).

15. Computer program product comprising instructions which when executed on a processor of a system for steering a plurality of intersecting image planes of a beamforming ultrasound imaging probe based on ultrasound signals detected by an ultrasound sensor disposed within a field of view of the probe, cause the processor to carry out the method steps of claim 14.
